# FASCICULE DE BREVET EUROPEEN

(11) **EP 2 571 432 B1**
(45) Date de publication et mention de la délivrance du brevet: **13.08.2014**
(21) Numéro de dépôt: 11727781.4
(22) Date de dépôt: 20.05.2011
(51) Int. Cl.: A61B 17/29, A61B 17/00

(54) **PINCE CHIRURGICALE A PHLEBECTOMIE**
CHIRURGISCHE PINZETTE FÜR PHLEBEKTOMIE
SURGICAL FORCEPS FOR PHLEBECTOMY

(30) Priorité: 21.05.2010 FR 1002161
(43) Date de publication de la demande: 27.03.2013
(73) Titulaire: Sarradon, Pierre, 83500 La Seyne-sur-Mer (FR)
(72) Inventeur: Sarradon, Pierre, 83500 La Seyne-sur-Mer (FR)
(74) Mandataire: Bugnion Genève
(86) Numéro de dépôt international: PCT/IB2011/052208
(87) Numéro de publication internationale: WO 2011/145078

(56) Documents cités:
- US-A- 5 234 460
- US-A- 5 817 128
- US-A1- 2006 079 933
- US-A1- 2008 046 001

## Description

### DOMAINE TECHNIQUE DE L'INVENTION

L'invention concerne le domaine des instruments chirurgicaux et notamment les instruments utilisés manuellement par un praticien pour saisir des tissus corporels.

L'invention concerne plus particulièrement une pince chirurgicale à phlébectomie.

### ÉTAT DE LA TECHNIQUE

La phlébectomie est une opération chirurgicale, concernant surtout le membre inférieur, qui consiste à retirer des segments de veines variqueuses, ces veines variqueuses étant inesthétiques, douloureuses et s'accompagnant d'une altération de l'état circulatoire présentant des risques de complication.

Pour permettre cette extraction, le chirurgien pratique une incision cutanée sur le patient. Par la suite, il saisit la veine au moyen d'un crochet connu sous le nom de « crochet de Muller » puis assure son extraction par des pinces de type « Halsted » ou équivalent, introduites dans l'incision.

Toutefois, l'utilisation de ce type de pinces présente l'inconvénient de nécessiter une incision relativement large, c'est-à-dire d'au moins 4 à 5 mm. En effet, la largeur des mors et surtout des branches nécessite une large incision. En particulier, l'écartement des branches lorsque la pince est ouverte est relativement important et majore la taille nécessaire des incisions.

En outre, plus le rayon d'action de la pince est élevé et plus l'écart entre les branches est important. Ainsi, lorsque les segments de veine à extraire se trouvent à une distance importante de l'incision, le praticien doit utiliser une pince présentant une longueur importante et l'incision pratiquée doit être d'autant plus large. Ainsi, en pratique, le praticien préfère réaliser de nombreuses incisions de sorte à limiter les distances entre les incisions et les segments de veine à extraire et, par conséquent, la largeur des incisions.

Par ailleurs, ces pinces sont peu ergonomiques. D'une part, elles ne permettent pas une préhension suffisante des segments de veine de sorte que le praticien doit parfois s'y reprendre à plusieurs fois pour les extraire. D'autre part, la manipulation des branches de manoeuvre à proximité de la peau du patient est peu aisée.

US 5 234 460 A montre une pince chirurgicale pour laparoscopie comportant un corps comprenant une platine d'articulation et une gaine tubulaire allongée, une mâchoire montée à l'extrémité distale de la gaine tubulaire et présentant deux mors dentés et deux branches de manoeuvre mobiles l'une par rapport à l'autre entre une position ouverte et une position rapprochée, chacune de ces branches comportant un anneau pour l'introduction d'un doigt, les anneaux sont écartés d'une distance d'au moins 1 centimètre par rapport à un plan de travail. Cette pince n'est pas destinée à une utilisation pour l'extraction de veines variqueuse et n'est donc pas optimisée pour ce type d'utilisation.

### OBJET DE L'INVENTION

L'invention vise à remédier à ces problèmes en proposant une pince chirurgicale à phlébectomie ergonomique et permettant de diminuer la taille et le nombre d'incisions nécessaires pour extraire l'intégralité des veines variqueuses.

À cet effet, et selon un premier aspect, l'invention propose une pince chirurgicale pour l'extraction de veines variqueuses comportant :
- un corps comprenant une platine d'articulation et une gaine tubulaire allongée ;
- deux branches de manoeuvre montées sur la platine d'articulation et mobiles l'une par rapport à l'autre entre une position ouverte et une position rapprochée, chacune de ces branches comportant un anneau pour l'introduction d'un doigt ;
- une mâchoire montée à l'extrémité distale de la gaine tubulaire et présentant deux mors montés mobiles l'un par rapport à l'autre autour d'un axe A entre une position ouverte et une position de préhension, les faces internes desdits mors étant dentées afin d'assurer le maintien des veines variqueuses ; et
- des moyens pour transmettre le mouvement des branches de manoeuvre aux mors, au travers de la gaine tubulaire, agencés pour déplacer les mors vers leur position de préhension lorsque les branches de manoeuvre sont rapprochées l'une de l'autre.

Ainsi, lorsque la pince est positionnée en position d'extraction de la veine, c'est la gaine tubulaire qui est disposée au niveau de l'incision. Or, la gaine présente une section limitée qui, en outre, ne varie pas en fonction de la position des branches de manoeuvre ou des mors. Ainsi, la section de la gaine tubulaire étant restreinte, la largeur de l'incision peut être limitée.

En outre, la présence d'un dispositif de transmission du mouvement des branches de manoeuvre aux mors, au travers de la gaine, permet d'augmenter le rayon d'action de la pince sans pour autant augmenter la largeur de l'incision. Dès lors, la pince selon l'invention permet d'extraire dans les mêmes conditions, des veines, que celles-ci soient près ou loin de l'incision. Aussi, le nombre d'incisions pratiquées lors d'une intervention peut être sensiblement diminué.

Les anneaux sont écartés d'une distance d'au moins 1 centimètre par rapport à un plan de travail, orthogonal à l'axe A, dans lequel s'étendent les mors. Cette réalisation permet de faciliter la manoeuvre de la pince car les anneaux sont ainsi écartés du patient de sorte à ménager un espace libre entre le patient et les anneaux de manoeuvre.

Les mors sont munis à leur extrémité distale d'une ou plusieurs griffes. Ces griffes permettent un ancrage du mors dans la paroi veineuse de sorte à permettre une extraction aisée des veines.

Avantageusement, les anneaux s'étendent dans un plan de manoeuvre incliné d'un angle supérieur à 10 ° par rapport audit plan de travail et, de préférence, incliné d'un angle compris entre 20 et 30 ° par rapport au plan de travail.

Avantageusement, la longueur de la gaine tubulaire est comprise entre 10 et 25 centimètres. De préférence, la gaine tubulaire présente une section inférieure ou égale à 3 millimètres. Ainsi, la pince tubulaire selon l'invention permet d'extraire des veines situées à plus de 25 centimètres de l'incision sans nécessiter une incision présentant une largeur supérieure à 3 millimètres.

Avantageusement, les faces internes de mors présentent des surfaces dentées de formes complémentaires. Cette disposition permet un maintien satisfaisant des segments de veines dans la mâchoire lors de l'opération.

Selon un mode de réalisation de l'invention, les moyens pour transmettre le mouvement comportent un arbre mobile en translation à l'intérieur de la gaine tubulaire et deux biellettes comportant chacune une première extrémité montée en rotation sur l'une des branches et une seconde extrémité montée en rotation sur une extrémité de l'arbre. Ainsi, ces moyens de transmission sont particulièrement simples.

Avantageusement, un premier mors est monté mobile par rapport à un second mors fixe, autour de l'axe A et l'extrémité distale de l'arbre mobile est équipée d'une biellette qui est d'une part montée mobile sur ledit premier mors autour d'un second axe B non confondu avec l'axe A et d'autre part montée en rotation sur l'extrémité distale de l'arbre.

Selon un second aspect, l'invention concerne un ensemble comportant un jeu des pinces chirurgicales selon le premier aspect de l'invention, chacune desdites pinces comprenant des gaines tubulaires de longueurs différentes.

### BRÈVE DESCRIPTION DES FIGURES

D'autres objets et avantages de l'invention apparaîtront au cours de la description qui suit, faite en référence aux dessins annexés, dans lesquels :
- la figure 1 est une vue schématique d'une pince chirurgicale ;
- la figure 2 est une vue schématique latérale de la pince chirurgicale de la figure 1 ;
- la figure 3 est une vue détaillée de la mâchoire de la figure 1 ;
- la figure 4 représente une pince chirurgicale selon un mode de réalisation ;
- la figure 5 est une vue détaillée de la portion de manoeuvre de la pince chirurgicale de la figure 4 ;
- la figure 6 est une vue détaillée du mors fixe et de l'extrémité distale de la gaine tubulaire de la pince chirurgicale de la figure 4 ; et
- la figure 7 est une vue détaillée de la structure du mors mobile de la pince de la figure 4.

### EXEMPLES DE RÉALISATION

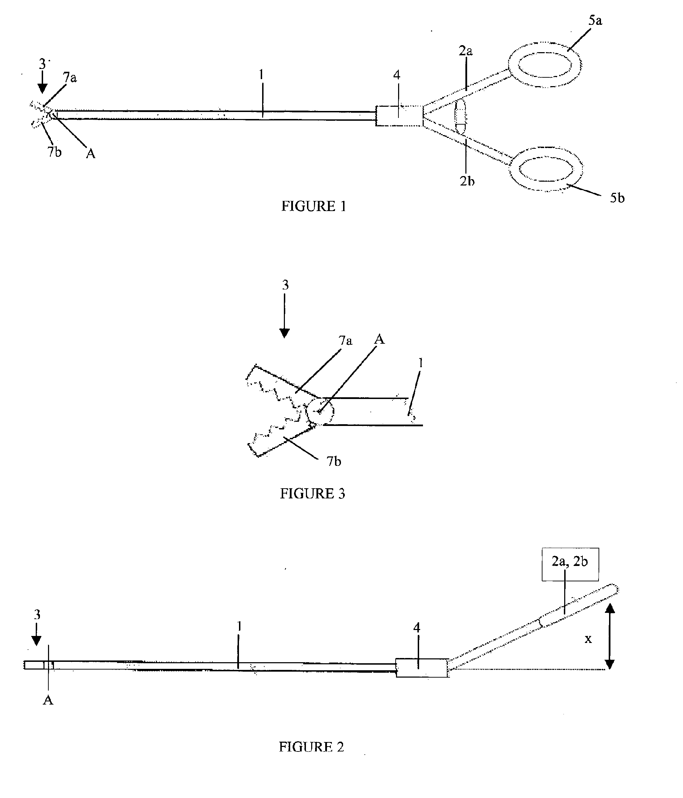

Une pince, telle que représentée sur les figures 1, 2 et 4, comporte un corps 1, deux branches 2a, 2b de manoeuvre et une mâchoire 3 de préhension des veines.

Le corps comporte une platine d'articulation 4 et une gaine tubulaire 1 dont l'extrémité distale porte la mâchoire 3. La gaine tubulaire 1 présente une longueur comprise entre 10 et 25 centimètres en fonction du rayon d'action de la pince souhaité et une section inférieure ou égale à 3 millimètres.

Deux branches 2a, 2b de manoeuvre sont montées, sur la platine d'articulation 4, mobiles entre une position ouverte et une position rapprochée.

Par ailleurs, la mâchoire 3 comporte deux mors 7a, 7b qui sont montés mobiles l'un par rapport à l'autre autour d'un axe A entre une position ouverte et une position de préhension.

Des moyens, décrits de manière détaillée par la suite, permettent de transmettre le mouvement des branches de manoeuvre 2a, 2b à la mâchoire 3 au travers de la gaine tubulaire 1. Ces moyens sont agencés de telle sorte que le rapprochement des branches 2a, 2b de manoeuvre entraînent le mouvement des mors 7a, 7b vers leur position de préhension et l'écartement des branches 2a, 2b entraînent le mouvement des mors 7a, 7b vers leur position écartée.

Les mors 7a, 7b s'étendent dans un plan, orthogonal à l'axe A de pivotement desdits mors 7a, 7b, qui sera appelé « plan de travail » par la suite. La gaine tubulaire 1 s'étend également dans ce plan de travail.

Les branches 2a, 2b de manoeuvre sont montées sur la platine d'articulation 4 et sont mobiles l'une par rapport à l'autre entre une position ouverte et une position rapprochée. Dans les modes de réalisation illustrés, les deux branches 2a, 2b sont montés mobiles sur la platine d'articulation 4 et pivotent autour d'un même axe de pivotement. Dans un autre mode de réalisation de l'invention, on peut également prévoir que l'une des branches 2a, 2b soit fixe par rapport à la platine d'articulation 4 alors que l'autre branche 2b, 2a est monté mobile en rotation par rapport à ladite branche fixe 2a, 2b.

Afin de faciliter la manoeuvre de la pince, les anneaux 5a, 5b sont écartés par rapport au plan de travail. De préférence, les anneaux 5a, 5b sont écartés, par rapport audit plan de travail, d'une distance x égale ou supérieure à un centimètre (voir figure 2). Cette disposition permet d'améliorer l'ergonomie de la pince.

Dans les modes de réalisation représentés, les anneaux 5a, 5b s'étendent selon un plan de manoeuvre qui présente une angulation supérieure à 10 ° par rapport au plan de travail. De préférence, le plan de manoeuvre est incliné d'un angle compris entre 20 et 30° par rapport audit plan de travail. L'angulation permet de décaler les anneaux 5a, 5b du plan de la peau et donc de faciliter la saisie des anneaux et la manipulation des anneaux 5a, 5b par rapport à une réalisation dans laquelle les anneaux s'étendrait dans le plan de la gaine tubulaire 1. En effet, les veines variqueuses étant sous sous-cutanées, la pince est introduite sous la peau dans un plan sensiblement parallèle à la surface de la peau.

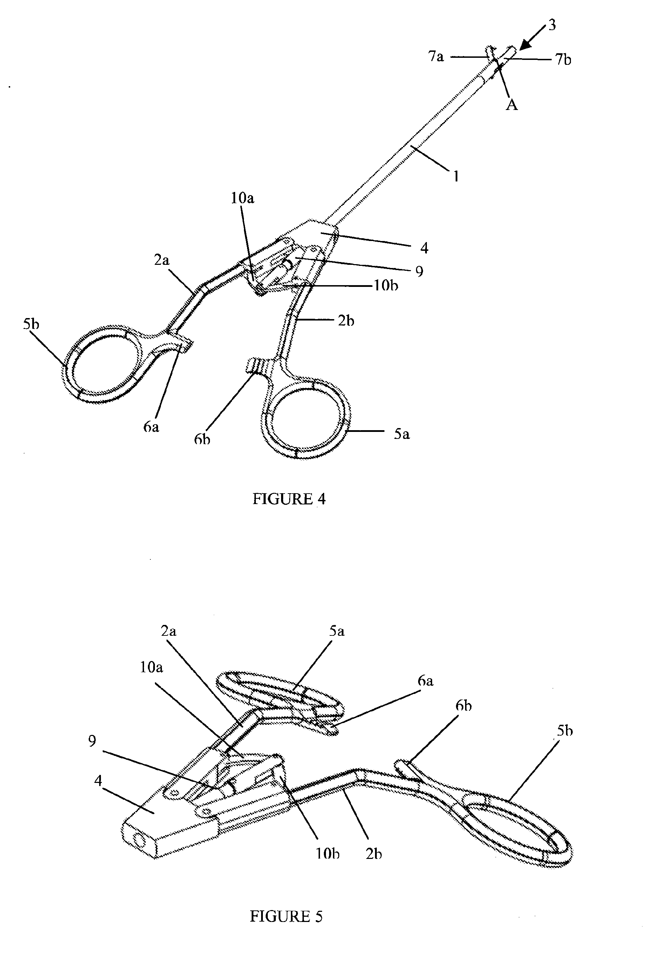

Selon un premier mode de réalisation représenté sur la figure 2, les branches 2a, 2b sont sensiblement longilignes et s'étendent dans un plan de manoeuvre incliné par rapport au plan de travail. L'axe de pivotement des branches 2a, 2b est dans ce cas orthogonal au plan de manoeuvre. Selon un second mode de réalisation représenté sur les figures 4 et 5, les branches 2a, 2b sont coudées et l'axe de pivotement des branches 2a, 2b est alors orthogonal au plan de travail.

Les branches 2a, 2b peuvent en outre être pourvues de moyens de blocage, représentés sur les figures 4 et 5, se présentant sous forme de pattes 6a, 6b pourvues de cliquets. Chacune des branches 2a, 2b porte un patte 6a, 6b orientée vers la patte de l'autre branche 2b, 2a. Les pattes 2a, 2b présentent deux surfaces munies de cliquets coopérant l'une contre l'autre lorsque les branches 2a, 2b sont en position rapprochée. Ces surfaces exercent une fonction de crémaillère et permettent de maintenir les mors fermés et sous-pression.

Par ailleurs, les mors 7a, 7b de la mâchoire 3 présentent une face interne pourvues de dents afin d'assurer une préhension satisfaisante des segments de veines. De manière avantageuse, les faces internes des mors présentent des formes complémentaires.

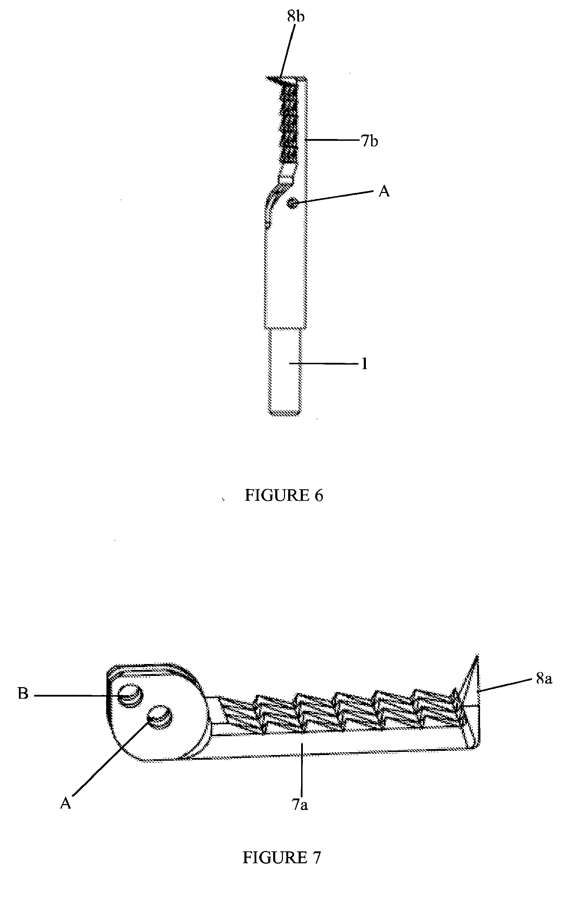

Les figures 6 et 7 représentent des surfaces internes de mors 7a, 7b. La surface interne comporte une pluralité de dents. De préférence, les motifs dentées de la surface interne s'étendent à la fois selon les directions longitudinale et latérale des mors 7a, 7b.

Par ailleurs, l'extrémité distale de chaque mors 7a, 7b est pourvue d'une griffe 8a, 8b ou crochet permettant d'assurer une meilleure préhension des veines variqueuses. Dans un mode de réalisation avantageux, un des mors 7a, 7b est équipé de deux griffes 8a, 8b alors que l'autre mors 7a, 7b est équipé d'au moins une griffe 8a, 8b venant s'insérer entre les deux griffes 8a, 8b du premier mors 7a, 7b,.

Dans le mode de réalisation représenté sur les figures 1 à 3, les deux mors 7a, 7b sont montés mobiles autour de l'axe A de pivotement.

Au contraire, dans le mode de réalisation représenté sur les figures 4, 6 et 7, la mâchoire 3 comporte un mors fixe 7b, représenté sur la figure 6, et un mors mobile 7a représenté sur la figure 7. Le mors fixe 7b s'étend sensiblement dans le prolongement de la gaine tubulaire 1 et le mors mobile 7a est monté en rotation, sur le mors fixe 7b, autour de l'axe de rotation A.

Dans le mode de réalisation représenté, les moyens de transmission du mouvement comportent un arbre 9 mobile en translation à l'intérieur de la gaine tubulaire 1. À une première extrémité dudit arbre 9, représenté sur les figures 4 et 5, les moyens de transmission comportent deux biellettes 10a, 10b. Chacune de ces biellettes 10a, 10b comporte une première extrémité montée en rotation sur l'une des branches 2a, 2b et une seconde extrémité montée en rotation sur la première extrémité de l'arbre 9. Les biellettes 10a, 10b sont agencées de telle sorte que lorsque les branches 2a, 2b sont rapprochées l'une de l'autre, l'arbre 9 coulisse vers l'extérieur de la gaine 1 et lorsque les branches 2a, 2b sont éloignées l'une de l'autre, l'arbre coulisse vers l'intérieur de la gaine 1.

La seconde extrémité de l'arbre 9, non représentée, est équipée d'une biellette qui est d'une part montée articulée sur l'arbre 9 et d'autre part montée articulée sur le mors mobile 7a. Cette biellette est montée articulée sur le mors mobile 7 autour d'un axe de levier B non confondu avec l'axe A d'articulation dudit mors 7a. L'axe de levier B est déterminé de telle sorte qu'une traction sur le mors mobile exercée au niveau dudit axe de levier B provoque la rotation du mors mobile 7a autour de l'axe A.

Bien évidemment, l'invention ne se limite pas à ces moyens particuliers de transmission du mouvement et l'on pourra également prévoir tout autres moyens appropriés sans sortir du cadre de l'invention. À titre d'exemple, on pourra également prévoir de transmettre le mouvement au moyen de câbles de traction-poussée ou équivalent.

Par ailleurs, la pince selon l'invention peut également être équipée de moyens de rappel, non représenté, agencés pour rappeler les branches 2a, 2b de manoeuvre vers leur position écartée. Ces moyens de rappels peuvent notamment être constitués d'un ou des plusieurs ressorts.

La pince à phlébectomie est par exemple réalisée en acier inoxydable, en titane, en carbone, en plastique ou dans une combinaison de ces matériaux, par exemple en associant l'acier et la plastique pour une réalisation à usage unique. De manière générale, tout matériaux utilisables en chirurgie peut être envisagé pour la fabrication d'une pince à phlébectomie selon l'invention. Pour des pinces destinées à être stérilisés, on choisira des matériaux supportant une stérilisation par autoclave.

Il est aussi possible d'avoir un jeu de pinces dont les gaines tubulaires présentent différentes tailles. Ainsi, le praticien pourra choisir la pince dont la longueur est la plus adaptée en fonction de la distance entre le segment de veine à extraire et l'incision.

L'invention est décrite dans ce qui précède à titre d'exemple. Il est entendu que l'homme du métier est à même de réaliser différentes variantes de réalisation de l'invention sans pour autant sortir du cadre de l'invention définit par les revendications.

## Revendications

1. Pince chirurgicale pour l'extraction de veines variqueuses comportant :
- un corps comprenant une platine d'articulation (4) et une gaine tubulaire allongée (1) ;
- une mâchoire (3) montée à l'extrémité distale de la gaine tubulaire (1) et présentant deux mors (7a, 7b) montés mobiles l'un par rapport à l'autre autour d'un axe A entre une position ouverte et une position de préhension, les faces internes desdits mors (7a, 7b) étant dentées afin d'assurer le maintien des veines variqueuses, les mors (7a, 7b) étant munis à leur extrémité distale d'une ou plusieurs griffe (8a, 8b) ;
- deux branches (2a, 2b) de manoeuvre montées sur la platine d'articulation (4) et mobiles l'une par rapport à l'autre entre une position ouverte et une position rapprochée, chacune de ces branches (2a, 2b) comportant un anneau (5a, 5b) pour l'introduction d'un doigt, les anneaux (5a, 5b) étant écartés d'une distance d'au moins 1 centimètre par rapport à un plan de travail, orthogonal à l'axe A, et dans lequel s'étendent les mors (7a, 7b); et
- des moyens pour transmettre le mouvement des branches (2a, 2b) de manoeuvre aux mors (7a, 7b), au travers de la gaine tubulaire (1), agencés pour déplacer les mors (7a, 7b) vers leur position de préhension lorsque les branches (2a, 2b) de manoeuvre sont rapprochées l'une de l'autre.

2. Pince chirurgicale selon la revendication 1, **caractérisée en ce que** les anneaux (5a, 5b) s'étendent dans un plan de manoeuvre incliné d'un angle supérieur à 10 ° par rapport audit plan de travail

3. Pince chirurgicale selon la revendication 2, **caractérisée en ce que** les anneaux (5a, 5b) s'étendent dans un plan de manoeuvre incliné d'un angle compris entre 20 et 30 ° par rapport au plan de travail.

4. Pince chirurgicale selon l'une quelconque des revendications 1 à 3, **caractérisée en ce que** la longueur de la gaine tubulaire (1) est comprise entre 10 et 25 cm.

5. Pince chirurgicale selon l'une quelconque des revendications 1 à 4, **caractérisée en ce que** la gaine tubulaire (1) présente une section inférieure ou égale à 3 millimètres.

6. Pince chirurgicale selon l'une des revendications 1 à 5, **caractérisée en ce que** les faces internes de mors (7a, 7b) présentent des surfaces dentées de formes complémentaires.

7. Pince chirurgicale selon l'une des revendications 1 à 6, **caractérisée en ce que** les moyens pour transmettre le mouvement comportent un arbre (9) mobile en translation à l'intérieur de la gaine tubulaire (1) et au moins une biellette (10a, 10b) comportant une première extrémité montée en rotation sur l'une des branches (2a, 2b) et une seconde extrémité montée en rotation sur une extrémité de l'arbre (9).

8. Pince chirurgicale selon la revendication 7, **caractérisée en ce qu'**un premier mors (8a) est monté mobile par rapport à un second mors (8b) fixe, autour de l'axe A et **en ce que** l'extrémité distale de l'arbre mobile (9) est équipée d'une biellette qui est d'une part montée mobile sur ledit premier mors (8a) autour d'un second axe B non confondu avec l'axe A et d'autre part montée en rotation sur l'extrémité distale de l'arbre (9).

9. Ensemble comportant un jeu de pinces chirurgicales selon l'une des revendications 1 à 8, chacune desdites pinces comprenant des gaines tubulaires (1) de longueurs différentes.

## Patentansprüche

1. Chirurgische Pinzette zum Entfernen von Krampfadern, die aufweist:
- einen Körper, der eine Gelenkplatte (4) und ein längliches Hüllrohr (1) umfasst,
- eine Klemme (3), die am distalen Ende des Hüllrohrs (1) montiert ist und zwei Backen (7a, 7b) aufweist, die zueinander um eine Achse A zwischen einer geöffneten Stellung und einer Greifstellung bewegbar montiert sind, wobei die Innenseiten der Backen (7a, 7b) gezahnt sind, um das Halten der Krampfadern sicherzustellen, wobei die Backen (7a, 7b) an ihrem distalen Ende mit einer oder mehreren Klauen (8a, 8b) ausgestattet sind,
- zwei Manövrierschenkel (2a, 2b), die auf der Gelenkplatte (4) montiert und zueinander zwischen einer geöffneten Stellung und einer angenäherten Stellung bewegbar sind, wobei jeder dieser Schenkel (2a, 2b) einen Ring (5a, 5b) zum Einführen eines Fingers aufweist, wobei die Ringe (5a, 5b) in einem Abstand von mindestens 1 Zentimeter im Verhältnis zu einer zur Achse A orthogonalen Arbeitsebene und in der sich die Backen (7a, 7b) erstrecken, abgespreizt sind, und
- Mittel zur Übertragung der Bewegung der Manövrierschenkel (2a, 2b) an die Backen (7a, 7b) durch das Hüllrohr (1), die ausgebildet sind, um die Backen (7a, 7b) in ihre Greifstellung zu verlagern, wenn die Manövrierschenkel (2a, 2b) einander angenähert sind.

2. Chirurgische Pinzette nach Anspruch 1, **dadurch gekennzeichnet, dass** sich die Ringe (5a, 5b) in einer Manövrierebene erstrecken, die im Verhältnis zur Arbeitsebene in einem Winkel größer als 10° geneigtist.

3. Chirurgische Pinzette nach Anspruch 3, **dadurch gekennzeichnet, dass** sich die Ringe (5a, 5b) in einer Manövrierebene erstrecken, die im Verhältnis zur Arbeitsebene in einem Winkel zwischen 20 und 30° inklusive geneigt ist.

4. Chirurgische Pinzette nach einem der Ansprüche 1 bis 3, **dadurch gekennzeichnet, dass** die Länge des Hüllrohrs (1) zwischen 10 und 25 cm ist.

5. Chirurgische Pinzette nach einem der Ansprüche 1 bis 4, **dadurch gekennzeichnet, dass** das Hüllrohr (1) einen Querschnitt kleiner oder gleich 3 Millimeter aufweist.

6. Chirurgische Pinzette nach einem der Ansprüche 1 bis 5, **dadurch gekennzeichnet, dass** die Innenseiten der Backen (7a, 7b) komplementär geformte gezahnte Flächen aufweisen.

7. Chirurgische Pinzette nach einem der Ansprüche 1 bis 6, **dadurch gekennzeichnet, dass** die Mittel zur Übertragung der Bewegung eine im Hüllrohr (1) verschiebend bewegbare Welle (9) und mindestens eine Pleuelstange (10a, 10b) aufweisen, die ein auf einem der Schenkel (2a, 2b) drehbar montiertes erstes Ende und ein auf einem Ende der Welle (9) drehbar montiertes zweites Ende aufweisen.

8. Chirurgische Pinzette nach Anspruch 7, **dadurch gekennzeichnet, dass** eine erste Backe (8a) im Verhältnis zu einer zweiten starren Backe (8b) um die Achse A bewegbar montiert ist und dass das distale Ende der bewegbaren Welle (9) mit einer Pleuelstange ausgestattet ist, die einerseits auf der ersten Backe (8a) um eine zweite Achse B, die nicht mit der Achse A zusammenfällt, bewegbar montiert ist und andererseits auf dem distalen Ende der Welle (9) drehbar montiert ist.

9. Gruppe, die ein Set chirurgischer Pinzetten nach einem der Ansprüche 1 bis 8 aufweist, wobei jede der Pinzetten Hüllrohre (1) unterschiedlicher Längen umfasst.

## Claims

1. A surgical forceps for removing varicose veins including:
- a body with an articulation plate (4) and an elongate tubular sheath (1);
- a gripper (3) mounted at the distal end of the tubular sheath (1) and having two jaws (7a, 7b) mounted movably relative to each other around an axis A between an open position and a gripping position, the inner faces of said jaws (7a, 7b) being toothed in order to hold the varicose veins, the jaws (7a, 7b) being provided at their distal end with one or more claws (8a, 8b);
- two maneuvering branches (2a, 2b) mounted on the articulation plate (4) and movable relative to each other between an open position and a closed position, each of these branches (2a, 2b) having a ring (5a, 5b) for the insertion of a finger, the rings (5a, 5b) being spaced apart by a distance of at least 1 cm relative to a working plane, orthogonal to the axis A, in which the jaws (7a, 7b) extend;
- means for transmitting the movement of the maneuvering branches (2a, 2b) to the jaws (7a, 7b), via the tubular sheath (1), said means being designed to move the jaws (7a, 7b) to their gripping position when the maneuvering branches (2a, 2b) are closed together.

2. The surgical forceps according to claim 1, **characterized in that** the rings (5a, 5b) extend in a maneuvering plane inclined by an angle greater than 10° relative to the working plane.

3. The surgical forceps according to claim 2, **characterized in that** the rings (5a, 5b) extend in a maneuvering plane inclined by an angle comprised between 20 and 30° relative to the working plane.

4. The surgical forceps according to any one of claims 1 to 3, **characterized in that** the length of the tubular sheath (1) is comprised between 10 and 25 cm.

5. The surgical forceps according to any one of claims 1 to 4, **characterized in that** the tubular sheath (1) has a section smaller than or equal to 3 mm.

6. The surgical forceps according to one of claims 1 to 5, **characterized in that** the inner faces of the jaws (7a, 7b) have toothed surfaces with complementary shapes.

7. The surgical forceps according to one of claims 1 to 6, **characterized in that** the means for transmitting the movement include a shaft (9) translatable inside the tubular sheath (1) and at least one connecting rod (10a, 10b) having a first end rotatably mounted on one of the branches (2a, 2b) and a second end rotatably mounted on one end of the shaft (9).

8. The surgical forceps according to claim 7, **characterized in that** a first jaw (8a) is movably mounted relative to a second stationary jaw (8b), around the axis A, and **in that** the distal end of the moving shaft (9) is equipped with a connecting rod that is on the one hand movably mounted on said first jaw (8a) around a second axis B not combined with the axis A, and on the other hand mounted rotatably on the distal end of the shaft (9).

9. An assembly including a set of surgical forceps according to one of claims 1 to 8, each of said forceps comprising tubular sheaths (1) of different lengths.
